# EUROPÄISCHE PATENTSCHRIFT

(11) **EP 2 998 726 B1**
(45) Veröffentlichungstag und Bekanntmachung des Hinweises auf die Patenterteilung: **25.01.2017**
(21) Anmeldenummer: 14185251.7
(22) Anmeldetag: 18.09.2014
(51) Int. Cl.: G01N 21/51, G01N 21/47

(54) **Verfahren und Vorrichtung zur nephelometrischen Bestimmung eines Analyten**
Method and device for nephelometric determination of an analyte
Procédé et dispositif de détermination néphélométrique d'un analyte

(43) Veröffentlichungstag der Anmeldung: 23.03.2016
(73) Patentinhaber: Siemens Healthcare Diagnostics Products GmbH, 35041 Marburg (DE)
(72) Erfinder: Pryshchepna, Oksana, 63456 Hanau (DE); Steinebach, Wolfgang, 56414 Salz (DE)

(56) Entgegenhaltungen:
- EP-A1- 2 309 251
- EP-A2- 0 997 726
- EP-A2- 1 091 205
- US-A- 4 549 809
- US-A- 6 096 561
- US-B1- 6 723 287

## Beschreibung

Die vorliegende Erfindung betrifft ein Verfahren zur nephelometrischen Bestimmung eines Analyten sowie ein Nephelometriesystem für ein automatisches Analysegerät.

Zahlreiche Nachweis- und Analyseverfahren zur Bestimmung physiologischer Parameter in Körperflüssigkeitsproben oder anderen biologischen Proben werden heute automatisiert in großer Anzahl in automatischen Analysegeräten, auch so genannten in vitro-Diagnostiksystemen, durchgeführt.

Heutige Analysegeräte sind in der Lage, eine Vielzahl von Nachweisreaktionen und Analysen mit einer Probe durchzuführen. Um eine Vielzahl von Untersuchungen automatisiert durchführen zu können, sind diverse Vorrichtungen zum räumlichen Transfer von Messzellen, Reaktionsbehältern und Reagenzbehältern erforderlich, wie z.B. Transferarme mit Greiffunktion, Transportbänder oder drehbare Transporträder, sowie Vorrichtungen zum Transfer von Flüssigkeiten, wie z.B. Pipettiervorrichtungen. Die Geräte umfassen eine Steuereinheit, die mittels entsprechender Software dazu in der Lage ist, die Arbeitsschritte für die gewünschten Analysen weitgehend selbstständig zu planen und abzuarbeiten.

Viele der in derartigen automatisiert arbeitenden Analysegeräten verwendeten Analyseverfahren beruhen auf optischen Methoden. Die Bestimmung klinisch relevanter Parameter, wie zum Beispiel der Konzentration oder Aktivität eines Analyten, erfolgt vielfach, indem ein Teil einer Probe mit einem oder mehreren Testreagenzien in einem Reaktionsgefäß, welches auch die Messzelle sein kann, vermischt wird, wodurch eine biochemische Reaktion oder eine spezifische Bindungsreaktion in Gang gesetzt wird, die eine messbare Veränderung einer optischen oder anderen physikalischen Eigenschaft des Testansatzes bewirkt.

Neben der Spektrophotometrie ist die Nephelometrie eine weit verbreitete Analysemethode. Mittels Nephelometrie lässt sich beispielsweise die Konzentration feinverteilter, kolloidaler Teilchen in Flüssigkeiten oder Gasen quantitativ bestimmen. Wird eine Suspension kleiner Partikel in einen Lichtstrahl verbracht, so wird ein Teil des eintretenden Lichtes absorbiert, ein anderer Teil, auch als Primärstrahl bezeichnet, verlässt die Suspension ungestreut und wieder ein anderer Teil wird seitlich zum eintretenden Strahl gestreut. Bei der Nephelometrie wird dieses seitlich austretende Streulicht gemessen.

Die Nephelometrie wird vor allem für den quantitativen oder qualitativen Nachweis von Analyten, beispielsweise Proteinen, eingesetzt, die mittels einer spezifischen Bindungsreaktion zwischen spezifischen Bindungspartnern, z.B. mittels Antigen-Antikörper-Bindung, nachweisbar sind.

Ein Nephelometriesystem umfasst mindestens eine Lichtquelle, mindestens einen Fotodetektor sowie mindestens eine Aufnahmeposition für eine Messzelle. Typischerweise ist die Anordnung von Lichtquelle und Lichtdetektor so gewählt, dass das Streulicht messbar ist, das von in der Probe nachzuweisenden Makromolekülen, beispielsweise von Partikelaggregaten, die infolge einer Analyt-abhängigen Reaktion in einem Reaktionsansatz entstehen, gestreut wird.

Auf dem Markt existieren unterschiedliche Bauweisen, die sich in der Anordnung von Lichtquelle, Aufnahmeposition für die Messzelle und Fotodetektor unterscheiden. Beispielsweise kann in einer Bauweise der Fotodetektor seitlich vom von der Lichtquelle ausgesandten Lichtstrahl angeordnet sein, um das gestreute Licht in einem Winkelbereich von 90° zur Richtung des von der Lichtquelle ausgesandten Lichtstrahls zu erfassen. Dies hat den Vorteil, dass die Intensität des Streulichts relativ niedrig sein kann und die Beeinflussung der Messung durch den von der Lichtquelle ausgesandten nicht gestreuten Teil des Lichtstrahls, der als Primärstrahl bezeichnet wird, relativ gering wird.

In einer anderen Bauweise können Lichtquelle, Aufnahmeposition und Fotodetektor derart angeordnet sein, dass der Fotodetektor das gestreute Licht in Winkelbereichen um die Ausbreitungsrichtung des von der Lichtquelle ausgesandten Lichtstrahls erfasst, in denen die Intensität des Streulichts relativ hoch ist. Den Fotodetektor erreicht in dieser Geometrie jedoch nicht nur das Streulicht, sondern auch der Primärstrahl. Da jedoch nur der gestreute Teil des Lichtes zum Messergebnis beitragen soll, ist für eine Optimierung des Messergebnisses eine vollständige Ausblendung des Primärstrahls erforderlich.

Zur Ausblendung des Primärstrahls werden üblicherweise optische Blenden eingesetzt. Diese werden mittels dünner Befestigungen, wie z.B. Drähten im Strahlengang gehalten und hinsichtlich ihrer Größe und Form so angepasst, dass sie den Primärstrahl vorzugsweise vollständig ausblenden, so dass möglichst ausschließlich Streulicht auf den Detektor trifft. Vorzugsweise ist das Verhältnis von Streulichtanteilen zu Primärstrahlanteilen kleiner als 0,001.

Zunehmende Verbreitung finden Analysegeräte, bei denen entweder die optische Einheit relativ zu den Messzellen bewegbar ist oder bei denen die Messzellen relativ zur optischen Einheit bewegbar sind. Dies hat den Vorteil, dass mit einer optischen Einheit eine Vielzahl von Proben gewissermaßen gleichzeitig untersucht werden kann, was den Probendurchsatz signifikant erhöht.

In EP-A1-2309251 ist eine Vorrichtung für die photometrische Untersuchung von Proben beschrieben, bei der die Messzellen stationär ausgebildet und kreisbogenförmig angeordnet sind, während sich die optische Einheit kreisbogenförmig entlang der Messzellenanordnung bewegt.

Die EP-A2-0997726 offenbart ein Verfahren zur nephelometrischen Bestimmung eines Analyten in einer Probe gemäß dem Oberbegriff des Anspruchs 1 sowie ein Nephelometriesystem gemäß dem Oberbegriff des Anspruchs 4.

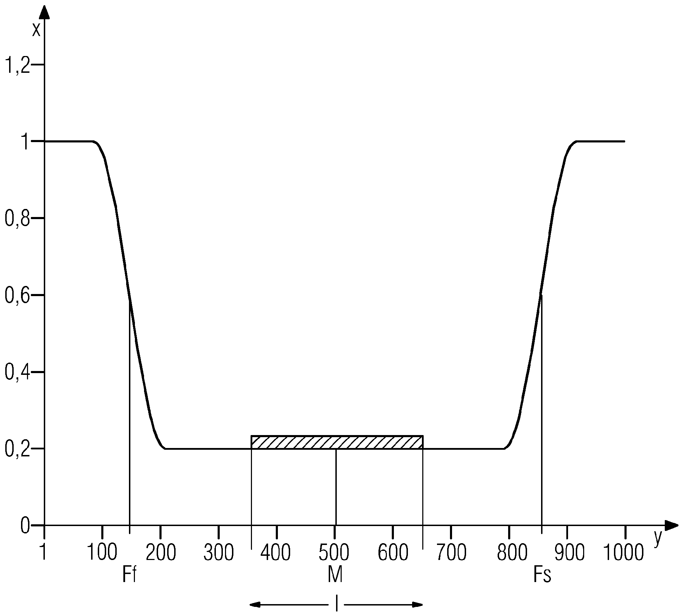

Bei derartigen optischen Systemen, bei denen die optische Einheit relativ zur Messzelle bewegt wird (oder umgekehrt), wandert der Lichtstrahl entlang einer Wegstrecke, vorzugsweise quer durch die Messzelle, und es werden mehrere Messwerte erfasst, wobei aufgrund der Bewegung jeder einzelne Messwert aus einer anderen Position der Messzelle stammt. Dadurch entsteht bei nephelometrischen Messungen eine typische, wannenförmige Kurve (siehe Figur 1) mit einer ersten, fallenden Flanke, einem Kurvenboden und einer zweiten, steigenden Flanke. Beim Eintritt (fallende Flanke) und beim Austritt (steigende Flanke) der Messzelle in bzw. aus dem Primärstrahl trifft das Licht des Primärstrahls auf die Messzellenwände, wird reflektiert oder gebrochen und wird an der Blende, die eigentlich den Primärstrahl ausblenden soll, vorbei geleitet auf den Fotodetektor. Der für die Analytbestimmung wesentliche Bereich liegt in Bereich des Kurvenbodens, wo die Ausblendung des Primärstrahls maximal ist. Ferner kommt es aufgrund von mechanischen Toleranzen der bewegten Komponenten bezüglich der Lage des Streuvolumens in der Messzelle zu einer Veränderung zwischen zeitlich aufeinander folgenden Messungen derselben Probe, was wichtig ist, wenn beispielsweise eine Reaktionskinetik erfasst werden soll.

Es ist daher für eine präzise Analytbestimmung notwendig, aus der Gesamtheit der gemessenen Lichtintensitätssignale diejenigen Lichtintensitätssignale für die weitere Auswertung auszuwählen, die aus dem gestreuten Anteil des Lichtstrahls nach Durchtritt durch die Messzelle resultieren und möglichst keinen Primärlichtanteil aufweisen.

Aufgabe der Erfindung ist es also, die Messqualität eines Nephelometriesystems zu verbessern, bei welchem die Lichtquelle, die Blende und der Fotodetektor einerseits und die Aufnahmeposition andererseits relativ zueinander bewegbar sind.

Die Aufgabe wird dadurch gelöst, dass aus der Gesamtheit der Lichtintensitätssignale, die während des Wanderns des Lichtstrahls entlang einer Wegstrecke durch die Messzelle aufgezeichnet werden, die Lage eines Intervalls I automatisch bestimmt wird, das ausschließlich Lichtintensitätssignale enthält, die aus dem gestreuten Anteil des Lichtstrahls nach Durchtritt durch die Messzelle resultieren und keinen oder nur einen unwesentlichen Primärlichtanteil aufweisen.

Gegenstand der vorliegenden Erfindung ist damit ein Verfahren zur nephelometrischen Bestimmung eines Analyten in einer Probe, wobei sich die Probe in einer Messzelle befindet. Das Verfahren umfasst die Schritte:
a. Platzieren der Messzelle in einem Nephelometriesystem mit mindestens einer optischen Einheit, die optische Einheit umfassend mindestens eine Lichtquelle zum Aussenden eines Lichtstrahls, eine Blende zum Ausblenden des ungestreuten Anteils des Lichtstrahls nach Durchtritt durch die Messzelle und einen Fotodetektor zum Empfang von gestreuten Anteilen des Lichtstrahls nach Durchtritt durch die Messzelle;
b. Bewegen der Messzelle und/oder Bewegen der optischen Einheit, so dass der von der Lichtquelle ausgesandte Lichtstrahl die Messzelle entlang einer Wegstrecke durchwandert;
c. Aufzeichnen der vom Fotodetektor empfangenen Lichtintensitätssignale über die Wegstrecke, in der der von der Lichtquelle ausgesandte Lichtstrahl die Messzelle durchwandert;
d. Bestimmen der Lage eines Intervalls I der aufgezeichneten Lichtintensitätssignale, das ausschließlich Lichtintensitätssignale enthält, die aus dem gestreuten Anteil des Lichtstrahls nach Durchtritt durch die Messzelle resultieren, wobei sich die Größe des Intervalls I aus einer definierten Anzahl von Lichtintensitätssignalen ergibt und ein für das verwendete Nephelometriesystem vorbestimmter Parameter ist; und
e. Bestimmen des Analyten anhand eines Lichtintensitätssignals oder anhand eines Mittelwertes mehrerer Lichtintensitätssignale aus dem Intervall I der aufgezeichneten Lichtintensitätssignale.

Die Lage des Intervalls I der aufgezeichneten Lichtintensitätssignale wird ermittelt, indem die über die Wegstrecke aufgezeichneten Lichtintensitätssignale wie folgt ausgewertet werden:
- Bilden der ersten und der zweiten Ableitung der über die Wegstrecke aufgezeichneten Lichtintensitätssignale;
- Bestimmen einer ersten Position Ff in der Wegstrecke mit den Bedingungen f'(x) < 0 und f" (x) = 0;
- Bestimmen einer zweiten Position Fs in der Wegstrecke mit den Bedingungen f'(x) > 0 und f" (x) = 0; und
- Bestimmen einer dritten Position M in der Wegstrecke durch Anwendung der Formel M = Ff + (Fs - Ff)/2; und
- Positionierung des Intervalls I, so dass die Position M die Mitte des Intervalls I bildet.

Die Größe des Intervalls I ergibt sich aus einer definierten Anzahl von Lichtintensitätssignalen und ist ein für das verwendete Nephelometriesystem vorbestimmter Parameter. Der Parameter ist abhängig von der Größe, der Geometrie und des Materials der Messzelle, von der Geschwindigkeit, mit der der Primärstrahl quer durch die Messzelle bewegt wird, von der Größe, Geometrie und Anordnung der Blende zum Ausblenden des des ungestreuten Anteils des Lichtstrahls, von der Anzahl der Lichtintensitätssignale, die während eines Durchlaufs aufgezeichnet werden etc. Für ein gegebenes Nephelometriesystem ist daher empirisch zu ermitteln, wieviele aufeinanderfolgende Lichtintensitätssignale typischerweise bei einer Messung einer typischen Probe in einer typischen Messzelle erhalten werden, die ausschließlich aus dem gestreuten Anteil des Lichtstrahls nach Durchtritt durch die Probe/Messzelle resultieren, bei denen die Ausblendung des Primärstrahls also maximal ist. Bei der Festlegung der für das verwendete Nephelometriesystem spezifischen Größe des Intervalls I ist einerseits eine größtmögliche Anzahl von Lichtintensitätssignalen anzustreben, weil dies einen großen Signal-/Störabstand bewirkt; andererseits ist die Größe des Intervalls I so klein zu wählen, dass sichergestellt ist, dass weder der Anfangswert noch der Endwert des Intervalls I jemals im Bereich der fallenden oder steigenden Flanke der Signalkurve zu liegen kommt.

In einem beispielhaften Nephelometriesystem mit einer um kreisbogenförmig und stationär angeordnete Messzellen rotierenden optischen Einheit (Rotationsgeschwindigkeit 2 Hz) und mit Kunststoffmesszellen mit kreisrundem Querschnitt und einem Durchmesser von etwa 7 mm und einer Anzahl von etwa 1.000 Lichtintensitätssignalen, die während eines Durchlaufs des Lichtstrahls durch eine Messzelle aufgezeichnet werden, wurde in Vorversuchen beispielsweise ein Intervall I einer Größe von 300 Lichtintensitätssignalen ermittelt und für das Nephelometriessystem festgelegt.

Die erste Position Ff, die wie oben beschrieben bestimmt wird, entspricht dem Wendepunkt der fallenden Flanke der wannenförmigen Signalkurve.

Die zweite Position Fs, die wie oben beschrieben bestimmt wird, entspricht dem Wendepunkt der steigenden Flanke der wannenförmigen Signalkurve.

In einer Ausführungsform des Verfahrens werden die Verfahrensschritte b)-d) mindestens n-mal wiederholt, und das Bestimmen des Analyten in Schritt e) erfolgt anhand des Mittelwertes jeweils eines Lichtintensitätssignals aus den n+1 Intervallen I oder anhand eines Mittelwertes aus n+1 Mittelwerten mehrerer Lichtintensitätssignale aus den n+1 Intervallen I der aufgezeichneten Lichtintensitätssignale. n ist beispielsweise eine Zahl von 1 bis 50, vorzugsweise eine Zahl von 10-20. Die mehrfache Messung derselben Probe erhöht die Präzision der quantitativen Bestimmung des Analyten.

In einer anderen Ausführungsform des Verfahrens werden die Verfahrensschritte b)-d) mindestens n-mal wiederholt, und das Bestimmen des Analyten in Schritt e) erfolgt anhand der Veränderung jeweils eines Lichtintensitätssignals aus den n+1 Intervallen I über die Zeit oder anhand der Veränderung eines Mittelwertes mehrerer Lichtintensitätssignale aus den n+1 Intervallen I der aufgezeichneten Lichtintensitätssignale über die Zeit. n ist beispielweise eine Zahl von 1 bis 1000. Dies ermöglicht die Aufzeichnung einer Reaktionskinetik, deren Parameter, wie z.B. maximale Steigung, Fläche unter der Kurve etc. für die quantitative Bestimmung des Analyten herangezogen werden können.

Unter einer "Probe" ist eine Zusammensetzung zu verstehen, die den zu bestimmenden Analyten vermutlich enthält. In der in vitro-Diagnostik übliche Proben bestehen aus oder enthalten zumindest Blut, Plasma, Serum, Urin, Speichel, Liquor, Ohrensekret, Nasensekret oder anderen Körperflüssigkeiten oder in einer Flüssigkeit aufgenommene Körpergewebeproben oder Zellen. Der Begriff "Probe" umfasst insbesondere auch Reaktionsansätze, also Mischungen der eigentlichen Probe mit einem oder mehreren Reagenzien, z.B. Antikörper-beschichtete Latexpartikel, in denen die Menge oder Aktivität des Analyten anhand einer Veränderung einer optischen Eigenschaft bestimmt werden kann.

Ein weiterer Gegenstand der vorliegenden Erfindung ist ein Nephelometriesystem mit mindestens einer optischen Einheit, die mindestens eine Lichtquelle zum Aussenden eines Lichtstrahls, mindestens eine Aufnahmeposition für eine Messzelle, eine Blende zum Ausblenden des ungestreuten Anteils des Lichtstrahls nach Durchtritt durch eine in der Aufnahmeposition angeordnete Messzelle und einen Fotodetektor zum Empfang von gestreuten Anteilen des Lichtstrahls nach Durchtritt durch die Messzelle aufweist und wobei die Lichtquelle, die Blende und der Fotodetektor einerseits und die Aufnahmeposition andererseits relativ zueinander bewegbar sind. Ferner weist ein erfindungsgemäßes Nephelometriesystem eine Steuerung auf, die ein Verfahren mit den folgenden Verfahrensschritten steuert:
i. Bewegen der Messzelle und/oder Bewegen der optischen Einheit, so dass der von der Lichtquelle ausgesandte Lichtstrahl die Messzelle entlang einer Wegstrecke durchwandert;
ii. Aufzeichnen der vom Fotodetektor empfangenen Lichtintensitätssignale über dieWegstrecke, in der der von der Lichtquelle ausgesandte Lichtstrahl die Messzelle durchwandert;
iii. Bestimmen der Lage eines Intervalls I der aufgezeichneten Lichtintensitätssignale, das ausschließlich Lichtintensitätssignale enthält, die aus dem gestreuten Anteil des Lichtstrahls nach Durchtritt durch die Messzelle resultieren, wobei sich die Größe des Intervalls I aus einer definierten Anzahl von Lichtintensitätssignalen ergibt und ein für das verwendete Nephelometriesystem vorbestimmter Parameter ist; und
iv. Bestimmen eines Analyten anhand eines Lichtintensitätssignals oder anhand eines Mittelwertes mehrerer Lichtintensitätssignale aus dem Intervall I der aufgezeichneten Lichtintensitätssignale,
   wobei die Lage des Intervalls I der aufgezeichneten Lichtintensitätssignale ermittelt wird, indem die über die Wegstrecke aufgezeichneten Lichtintensitätssignale wie folgt ausgewertet werden:
   - Bilden der ersten und der zweiten Ableitung der über die Wegstrecke aufgezeichneten Lichtintensitätssignale;
   - Bestimmen einer ersten Position Ff in der Wegstrecke mit den Bedingungen f'(x) < 0 und f"(x) = 0;
   - Bestimmen einer zweiten Position Fs in der Wegstrecke mit den Bedingungen f'(x) > 0 und f"(x) = 0; und
   - Bestimmen einer dritten Position M in der Wegstrecke durch Anwendung der Formel M = Ff + (Fs - Ff)/2; und
   - Positionierung des Intervalls I, so dass die Position M die Mitte des Intervalls I bildet.

Eine bevorzugte Lichtquelle weist eine Laserdiode auf. Ebenso kann jedoch vorgesehen sein, dass es sich bei der Lichtquelle um eine Licht-emittierende Diode (LED), eine Glühlampe, eine Gasentladungslampe oder eine Lichtbogenlampe handelt. Vorteilhafterweise emittiert die Lichtquelle Licht in Wellenlängenbereichen zwischen 200 nm und 1400 nm, bevorzugt zwischen 300 und 1100 nm.

Bei dem Lichtdetektor handelt es sich bevorzugt um eine Photodiode, die sichtbares Licht, in manchen Ausführungen auch IR-, oder UV-Licht durch den inneren Photoeffekt in einen elektrischen Strom bzw. eine Spannung umwandelt. Dieser Prozess wird auch als Signalaufnahme bezeichnet, und der elektrische Strom bzw. die Spannung werden auch als Lichtintensitätssignal bezeichnet. Alternativ handelt es sich bei dem Lichtdetektor um einen CCD-Sensor. CCD-Sensoren bestehen aus einer Matrix oder einer Zeile mit lichtempfindlichen Photodioden. Ebenso kann jedoch vorgesehen sein, dass es sich bei dem Lichtdetektor um eine Photozelle, einen Siliziumphotodetektor, einen Avalanchephotodetektor oder einen Photomultiplier handelt.

Die Blende zum Ausblenden des Primärstrahls, d.h. des ungestreuten Anteils des Lichtstrahls nach Durchtritt durch die Messzelle, ist so angeordnet, dass der Primärstrahl absorbiert und/oder reflektiert wird.

Grundsätzlich kann die optische Einheit des erfindungsgemäßen Nephelometriesystems weiterhin noch Filter, Linsen, Spiegel oder andere optische Element aufweisen.

Bevorzugterweise weist ein erfindungsgemäßes Nephelometriesystem mindestens zwei, bevorzugt mit mindestens 16, besonders bevorzugt mit mindestens 32 Aufnahmepositionen für jeweils eine Messzelle auf.

Weiterhin bevorzugt sind die mindestens zwei Aufnahmepositionen für jeweils eine Messzelle in einer Kreisbahn angeordnet, und die Lichtquelle, die Blende und der Fotodetektor sind relativ zu den Aufnahmepositionen für jeweils eine Messzelle auf einer Kreisbahn bewegbar, oder die Aufnahmepositionen für die Messzellen sind relativ zu Lichtquelle, Blende und Fotodetektor auf einer Kreisbahn bewegbar.

Bevorzugterweise ist die mindestens eine Aufnahmeposition für die Aufnahme einer Messzelle mit ovalem oder rundem Querschnitt geeignet.

Ein weiterer Gegenstand der vorliegenden Erfindung ist ein automatisches Analysegerät, das ein erfindungsgemäßes Nephelometriesystem enthält.

Ein bevorzugtes automatisches Analysegerät umfasst ferner einen Behälter zur Aufnahme einer Vielzahl von Messzellen als Schüttgut, eine Vorrichtung zur Vereinzelung der Messzellen und eine Vorrichtung zur Positionierung einer einzelnen Messzelle in der mindestens einen Aufnahmeposition der optischen Einheit des Nephelometriesystems. Mit Hilfe eines solchen Analysegerätes ist eine vollautomatische Durchführung von nephelometrischen Analytbestimmungen in einer Vielzahl von Proben möglich.

### FIGURENBESCHREIBUNG

Figur 1 zeigt schematisch den typischen wannenförmigen Verlauf einer Lichtintensitätssignalkurve, die in einem Nephelometriesystem aufgezeichnet wurde, bei dem mehrere Aufnahmepositionen für jeweils eine Messzelle mit kreisrundem Durchmesser stationär auf einem Kreisbogen angeordnet sind und bei dem sich die optische Einheit, d.h. Lichtquelle, Blende und Fotodetektor auf einer Kreisbahn entlang der Aufnahmepositionen bewegt. Die Kurve zeigt die gemessene Lichtintensität (X-Achse) in Abhängigkeit von der Wegstrecke (Y-Achse), in der der Lichtstrahl eine Messzelle durchwandert. Die Kurve setzt sich aus 1.000 Messpunkten bzw. Lichtintensitätssignalen zusammen, die beim einmaligen Durchwandern des Lichtstrahls einer der Messzellen mit einer Probe, entlang der Wegstrecke aufgezeichnet wurden. In dem hier beispielhaft beschriebenen System beträgt der Abstand zwischen zwei Messpunkten 1,33 µm bzw. 1,06 µs. In der Realität sehen die so erhaltenen Kurven durch Störungen, Rauschen und Asymmetrien der Mechanik weniger ideal aus, allerdings können die erhaltenen Rohdaten einer gängigen Filterung zur Glättung der Kurve unterworfen werden. Es ist erkennbar, dass die Kurve eine erste, fallende Flanke, einen Kurvenboden und eine zweite, steigende Flanke aufweist. Der für die Analytbestimmung wesentliche Bereich liegt im Bereich des Kurvenbodens, wo die Ausblendung des Primärstrahls maximal ist. Mittels des erfindungsgemäßen Verfahrens werden der Wendepunkt Ff der fallenden Flanke und der Wendepunkt Fs der steigenden Flanke bestimmt. Dann wird der Punkt M gesucht, der genau in der Mitte der Punkte Ff und Fs liegt, und das für das hier verwendete Nephelometriesystem vorbestimmte Intervall I (hier 300 Lichtintensitätssignale/ Messpunkte groß), das ausschließlich Lichtintensitätssignale enthält, die aus dem gestreuten Anteil des Lichtstrahls nach Durchtritt durch die Messzelle resultieren, wird so positioniert, dass der Punkt M die Mitte des Intervalls I bildet. Die Auswertung der in dem Intervall I enthaltenen Lichtintensitätssignale ermöglicht eine präzise Bestimmung des Analyten.

## Patentansprüche

1. Verfahren zur nephelometrischen Bestimmung eines Analyten in einer Probe, wobei sich die Probe in einer Messzelle befindet, das Verfahren umfassend die Schritte:
a. Platzieren der Messzelle in einem Nephelometriesystem mit mindestens einer optischen Einheit, die optische Einheit umfassend mindestens eine Lichtquelle zum Aussenden eines Lichtstrahls, eine Blende zum Ausblenden des ungestreuten Anteils des Lichtstrahls nach Durchtritt durch die Messzelle und einen Fotodetektor zum Empfang von gestreuten Anteilen des Lichtstrahls nach Durchtritt durch die Messzelle;
b. Bewegen der Messzelle und/oder Bewegen der optischen Einheit, so dass der von der Lichtquelle ausgesandte Lichtstrahl die Messzelle entlang einer Wegstrecke durchwandert;
c. Aufzeichnen der vom Fotodetektor empfangenen Lichtintensitätssignale über die Wegstrecke, in der der von der Lichtquelle ausgesandte Lichtstrahl die Messzelle durchwandert;
d. Bestimmen der Lage eines Intervalls I der aufgezeichneten Lichtintensitätssignale, das ausschließlich Lichtintensitätssignale enthält, die aus dem gestreuten Anteil des Lichtstrahls nach Durchtritt durch die Messzelle resultieren, wobei sich die Größe des Intervalls I aus einer definierten Anzahl von Lichtintensitätssignalen ergibt und ein für das verwendete Nephelometriesystem vorbestimmter Parameter ist; und
e. Bestimmen des Analyten anhand eines Lichtintensitätssignals oder anhand eines Mittelwertes mehrerer Lichtintensitätssignale aus dem Intervall I der aufgezeichneten Lichtintensitätssignale,
**dadurch gekennzeichnet,**
**dass** die Lage des Intervalls I der aufgezeichneten Lichtintensitätssignale ermittelt wird, indem die über die Wegstrecke aufgezeichneten Lichtintensitätssignale wie folgt ausgewertet werden:
- Bilden der ersten und der zweiten Ableitung f'(x) und f"(x) der über die Wegstrecke aufgezeichneten Lichtintensitätssignale;
- Bestimmen einer ersten Position Ff in der Wegstrecke mit den Bedingungen f'(x) < 0 und f''(x) = 0;
- Bestimmen einer zweiten Position Fs in der Wegstrecke mit den Bedingungen f'(x) > 0 und f"(x) = 0; und
- Bestimmen einer dritten Position M in der Wegstrecke durch Anwendung der Formel M = Ff + (Fs - Ff)/2; und
- Positionierung des Intervalls I, so dass die Position M die Mitte des Intervalls I bildet.

2. Verfahren gemäß Anspruch 1, wobei die Verfahrensschritte b)-d) n-mal wiederholt werden und wobei das Bestimmen des Analyten in Schritt e) anhand des Mittelwertes jeweils eines Lichtintensitätssignals aus den n+1 Intervallen I oder anhand eines Mittelwertes aus n+1 Mittelwerten mehrerer Lichtintensitätssignale aus den n+1 Intervallen I der aufgezeichneten Lichtintensitätssignale erfolgt.

3. Verfahren gemäß Anspruch 1, wobei die Verfahrensschritte b)-d) n-mal wiederholt werden und wobei das Bestimmen des Analyten in Schritt e) anhand der Veränderung jeweils eines Lichtintensitätssignals aus den n+1 Intervallen I oder anhand der Veränderung eines Mittelwertes mehrerer Lichtintensitätssignale aus den n+1 Intervallen I der aufgezeichneten Lichtintensitätssignale über die Zeit erfolgt.

4. Nephelometriesystem mit mindestens einer optischen Einheit, die optische Einheit umfassend mindestens eine Lichtquelle zum Aussenden eines Lichtstrahls, mindestens eine Aufnahmeposition für eine Messzelle, eine Blende zum Ausblenden des ungestreuten Anteils des Lichtstrahls nach Durchtritt durch eine in der Aufnahmeposition angeordnete Messzelle und einen Fotodetektor zum Empfang von gestreuten Anteilen des Lichtstrahls nach Durchtritt durch die Messzelle, wobei die Lichtquelle, die Blende und der Fotodetektor einerseits und die Aufnahmeposition andererseits relativ zueinander bewegbar sind, **dadurch gekennzeichnet, dass** das Nephelometriesystem ferner eine Steuerung aufweist, die ein Verfahren mit den folgenden Verfahrensschritten steuert:
i. Bewegen der Messzelle und/oder Bewegen der optischen Einheit, so dass der von der Lichtquelle ausgesandte Lichtstrahl die Messzelle entlang einer Wegstrecke durchwandert;
ii. Aufzeichnen der vom Fotodetektor empfangenen Lichtintensitätssignale über die Wegstrecke, in der der von der Lichtquelle ausgesandte Lichtstrahl die Messzelle durchwandert;
iii. Bestimmen der Lage eines Intervalls I der aufgezeichneten Lichtintensitätssignale, das ausschließlich Lichtintensitätssignale enthält, die aus dem gestreuten Anteil des Lichtstrahls nach Durchtritt durch die Messzelle resultieren, wobei sich die Größe des Intervalls I aus einer definierten Anzahl von Lichtintensitätssignalen ergibt und ein für das verwendete Nephelometriesystem vorbestimmter Parameter ist; und
iv. Bestimmen eines Analyten anhand eines Lichtintensitätssignals oder anhand eines Mittelwertes mehrerer Lichtintensitätssignale aus dem Intervall I der aufgezeichneten Lichtintensitätssignale,
wobei die Lage des Intervalls I der aufgezeichneten Lichtintensitätssignale ermittelt wird, indem die über die Wegstrecke aufgezeichneten Lichtintensitätssignale wie folgt ausgewertet werden:
- Bilden der ersten und der zweiten Ableitung f'(x) und f"(x) der über die Wegstrecke aufgezeichneten Lichtintensitätssignale;
- Bestimmen einer ersten Position Ff in der Wegstrecke mit den Bedingungen f'(x) < 0 und f''(x) = 0;
- Bestimmen einer zweiten Position Fs in der Wegstrecke mit den Bedingungen f'(x) > 0 und f''(x) = 0; und
- Bestimmen einer dritten Position M in der Wegstrecke durch Anwendung der Formel M = Ff + (Fs - Ff)/2; und
- Positionierung des Intervalls I, so dass die Position M die Mitte des Intervalls I bildet.

5. Nephelometriesystem gemäß Anspruch 4, mit mindestens zwei, bevorzugt mit mindestens 16, besonders bevorzugt mit mindestens 32 Aufnahmepositionen für jeweils eine Messzelle.

6. Nephelometriesystem gemäß Anspruch 5, wobei die mindestens zwei Aufnahmepositionen für jeweils eine Messzelle in einer Kreisbahn angeordnet sind.

7. Nephelometriesystem gemäß einem der Ansprüche 4 bis 6, wobei die Lichtquelle, die Blende und der Fotodetektor relativ zu der mindestens einen Aufnahmeposition für eine Messzelle auf einer Kreisbahn bewegbar sind.

8. Nephelometriesystem gemäß einem der Ansprüche 4 bis 6, wobei die mindesntens eine Aufnahmeposition für eine Messzelle relativ zu Lichtquelle, Blende und Fotodetektor bewegbar ist.

9. Nephelometriesystem gemäß einem der Ansprüche 4 bis 8, wobei die mindestens eine Aufnahmeposition für die Aufnahme einer Messzelle mit ovalem oder rundem Querschnitt geeignet ist.

10. Automatisches Analysegerät enthaltend ein Nephelometriesystem gemäß einem der Ansprüche 4 bis 9.

11. Automatisches Analysegerät gemäß Anspruch 10, ferner umfassend einen Behälter zur Aufnahme einer Vielzahl von Messzellen als Schüttgut, eine Vorrichtung zur Vereinzelung der Messzellen und eine Vorrichtung zur Positionierung einer einzelnen Messzelle in der mindestens einen Aufnahmeposition der optischen Einheit des Nephelometriesystems.

## Claims

1. Method of nephelometric determination of an analyte in a sample, with the sample being situated in a measurement cell, the method comprising the following steps:
a. placing the measurement cell into a nephelometry system comprising at least one optical unit, the optical unit comprising at least one light source for emitting a light beam, a stop for blocking the non-scattered portion of the light beam after passing through the measurement cell and a photodetector for receiving scattered portions of the light beam after passing through the measurement cell;
b. moving the measurement cell and/or moving the optical unit such that the light beam emitted by the light source passes through the measurement cell along a route;
c. recording the light intensity signals received by the photodetector along the route, along which the light beam emitted by the light source travels through the measurement cell;
d. determining the location of an interval I of the recorded light intensity signals which only contains light intensity signals that emerge from the scattered portion of the light beam after passing through the measurement cell, wherein the size of the interval I emerges from a defined number of light intensity signals and is a predetermined parameter for the employed nephelometry system; and
e. determining the analyte on the basis of a light intensity signal or on the basis of a mean value for a plurality of light intensity signals from the interval I of the recorded light intensity signals,
**characterized**
**in that** the location of the interval I of the recorded light intensity signals is established by virtue of the light intensity signals recorded along the route being evaluated as follows:
- forming the first and second derivative f'(x) and f''(x) of the light intensity signals recorded along the route;
- determining a first position Ff along the route with the conditions f'(x) < 0 and f''(x) = 0;
- determining a second position Fs along the route with the conditions f'(x) > 0 and f''(x) = 0; and
- determining a third position M along the route by applying the formula M = Ff + (Fs - Ff)/2; and
- positioning the interval I such that the position M forms the center of the interval I.

2. Method according to Claim 1, wherein the method steps b)-d) are repeated n times and wherein determining the analyte in step e) is implemented on the basis of the mean value of respectively one light intensity signal from the n+1 intervals I or on the basis of a mean value from n+1 mean values of the plurality of light intensity signals from the n+1 intervals I of the recorded light intensity signals.

3. Method according to Claim 1, wherein the method steps b)-d) are repeated n times and wherein determining the analyte in step e) is implemented on the basis of the change in respectively one light intensity signal from the n+1 intervals I or on the basis of the change of a mean value of a plurality of light intensity signals from the n+1 intervals I of the recorded light intensity signals over time.

4. Nephelometry system comprising at least one optical unit, the optical unit comprising at least one light source for emitting a light beam, at least one receptacle position for a measurement cell a stop for blocking the non-scattered portion of the light beam after passing through a measurement cell arranged in the receptacle position and a photodetector for receiving scattered portions of the light beam after passing through the measurement cell, wherein the light source, the stop and the photodetector on the one hand and the receptacle position on the other hand are movable relative to one another, **characterized in that** the nephelometry system furthermore has a control which controls a method comprising the following method steps:
i. moving the measurement cell and/or moving the optical unit such that the light beam emitted by the light source passes through the measurement cell along a route;
ii. recording the light intensity signals received by the photodetector along the route, along which the light beam emitted by the light source travels through the measurement cell;
iii. determining the location of an interval I of the recorded light intensity signals which only contains light intensity signals that emerge from the scattered portion of the light beam after passing through the measurement cell, wherein the size of the interval I emerges from a defined number of light intensity signals and is a predetermined parameter for the employed nephelometry system; and
iv. determining an analyte on the basis of a light intensity signal or on the basis of a mean value for a plurality of light intensity signals from the interval I of the recorded light intensity signals,
wherein the location of the interval I of the recorded light intensity signals is established by virtue of the light intensity signals recorded along the route being evaluated as follows:
- forming the first and second derivative f'(x) and f''(x) of the light intensity signals recorded along the route;
- determining a first position Ff along the route with the conditions f'(x) < 0 and f''(x) = 0;
- determining a second position Fs along the route with the conditions f'(x) > 0 and f''(x) = 0; and
- determining a third position M along the route by applying the formula M = Ff + (Fs - Ff)/2; and
- positioning the interval I such that the position M forms the center of the interval I.

5. Nephelometry system according to Claim 4, comprising at least two, preferably comprising at least 16, particularly preferably comprising at least 32 receptacle positions for respectively one measurement cell.

6. Nephelometry system according to Claim 5, wherein the at least two receptacle positions for respectively one measurement cell are arranged along a circular path.

7. Nephelometry system according to one of Claims 4 to 6, wherein the light source, the stop and the photodetector are movable along a circular path relative to the at least one receptacle position for a measurement cell.

8. Nephelometry system according to one of Claims 4 to 6, wherein the at least one receptacle position for a measurement cell is movable relative to the light source, stop and photodetector.

9. Nephelometry system according to one of Claims 4 to 8, wherein the at least one receptacle position is suitable for receiving a measurement cell with an oval or round cross section.

10. Automatic analysis device, containing a nephelometry system according to one of Claims 4 to 9.

11. Automatic analysis device according to Claim 10, furthermore comprising a container for receiving a multiplicity of measurement cells as bulk material, an apparatus for separating the measurement cells and an apparatus for positioning a single measurement cell in the at least one receptacle position of the optical unit of the nephelometry system.

## Revendications

1. Procédé de détermination néphélométrique d'un analyte dans un échantillon, l'échantillon se trouvant dans une cellule de mesure, le procédé comprenant les stades :
a. placement de la cellule de mesure dans un système de néphélométrie ayant au moins une unité optique, l'unité optique comprenant au moins une source lumineuse pour l'émission d'un faisceau lumineux, un diaphragme pour diaphragmer la partie non dispersée du faisceau lumineux après traversée de la cellule de mesure et un photodétecteur de réception des parties dispersées du faisceau lumineux après traversée de la cellule de mesure ;
b. déplacement de la cellule de mesure et/ou déplacement de l'unité optique de manière à ce que le faisceau lumineux envoyé par la source lumineuse chemine dans la cellule de mesure le long d'une distance de cheminement ;
c. enregistrement des signaux d'intensité lumineuse reçus par le photodétecteur sur la distance de cheminement, sur laquelle le faisceau lumineux envoyé par la source lumineuse chemine dans la cellule de mesure ;
d. détermination de la position d'un intervalle I des signaux d'intensité lumineuse enregistrés, qui contient exclusivement des signaux d'intensité lumineuse, qui proviennent de la partie dispersée du faisceau lumineux après traversée de la cellule de mesure, la dimension de l'intervalle I étant obtenue à partir d'un nombre défini de signaux d'intensité lumineuse et étant un paramètre défini à l'avance pour le système de néphélométrie utilisé et
e. détermination de l'analyte à l'aide d'un signal d'intensité lumineuse ou à l'aide d'une valeur moyenne de plusieurs signaux d'intensité lumineuse à partir de l'intervalle I des signaux d'intensité lumineuse enregistrés,
**caractérisé**
**en ce que** l'on détermine la position de l'intervalle I des signaux d'intensité lumineuse enregistrés en exploitant de la manière suivante les signaux d'intensité lumineuse enregistrés sur la distance de cheminement :
- formation de la dérivée f'(x) première et de la dérivée f"(x) seconde des signaux d'intensité lumineuse enregistrés sur la distance de cheminement ;
- détermination d'une première position Ff dans la distance de cheminement avec les conditions f'(x) < 0 et f"(x) = 0 ;
- détermination d'une deuxième position Fs dans la distance de cheminement avec les conditions f'(x) > 0 et f''(x) = 0 et
- détermination d'une troisième position M dans la distance de cheminement en appliquant la formule M = Ff + (Fs - Ff)/2 et
- positionnement de l'intervalle I, de manière à ce que la position M forme le milieu de l'intervalle I.

2. Procédé suivant la revendication 1, dans lequel on répète n fois les stades b) à d) du procédé et dans lequel on effectue la détermination de l'analyte au stade e) à l'aide de la valeur moyenne respectivement d'un signal d'intensité lumineuse parmi les n+1 intervalles I ou à l'aide d'une valeur moyenne parmi n+1 valeurs moyennes de plusieurs signaux d'intensité lumineuse parmi les n+1 intervalles I des signaux d'intensité lumineuse enregistrés.

3. Procédé suivant la revendication 1, dans lequel on répète n fois les stades b) à d) et dans lequel on effectue la détermination de l'analyte au stade e) à l'aide de la modification d'un signal d'intensité lumineuse parmi les n+1 intervalles I ou à l'aide de la modification d'une valeur moyenne de plusieurs signaux d'intensité lumineuse parmi les n+1 intervalles I des signaux d'intensité lumineuse enregistrés en fonction du temps.

4. Système de néphélométrie comprenant au moins une unité optique, l'unité optique comprenant au moins une source lumineuse d'émission d'un faisceau lumineux, au moins une position de réception d'une cellule de mesure, un diaphragme pour diaphragmer la partie non dispersée du faisceau lumineux après passage dans une cellule de mesure mise en la position de réception et un photodétecteur de réception de parties dispersées du faisceau lumineux après passage dans la cellule de mesure, la source lumineuse, le diaphragme et le photodétecteur, d'une part, et la position de réception, d'autre part, étant mobiles l'un par rapport à l'autre, **caractérisé en ce que** le système de néphélométrie a, en outre, une commande, qui commande un procédé ayant les stades de procédé suivantes :
i. déplacement de la cellule de mesure et/ou déplacement de l'unité optique de manière à ce que le faisceau lumineux envoyé par la source lumineuse chemine dans la cellule de mesure le long d'une distance de cheminement ;
ii. enregistrement des signaux d'intensité lumineuse reçus par le photodétecteur sur la distance de cheminement, sur laquelle le faisceau lumineux envoyé par la source lumineuse chemine dans la cellule de mesure ;
iii. détermination de la position d'un intervalle I des signaux d'intensité lumineuse enregistrés, qui contient exclusivement des signaux d'intensité lumineuse, qui proviennent de la partie dispersée du faisceau lumineux après traversée de la cellule de mesure, la dimension de l'intervalle I étant obtenue à partir d'un nombre défini de signaux d'intensité lumineuse et étant un paramètre défini à l'avance pour le système de néphélométrie utilisé et
iv. détermination d'un analyte à l'aide d'un signal d'intensité lumineuse ou à l'aide d'une valeur moyenne de plusieurs signaux d'intensité lumineuse à partir de l'intervalle I des signaux d'intensité lumineuse enregistrés.
dans lequel on détermine la position de l'intervalle I des signaux d'intensité lumineuse enregistrés en exploitant de la manière suivante les signaux d'intensité lumineuse enregistrés sur la distance de cheminement :
- formation de la dérivée f'(x) première et de la dérivée f"(x) seconde des signaux d'intensité lumineuse enregistrés sur la distance de cheminement ;
- détermination d'une première position Ff dans la distance de cheminement avec les conditions f'(x) < 0 et f"(x) = 0 ;
- détermination d'une deuxième position Fs dans la distance de cheminement avec les conditions f'(x) > 0 et f''(x) = 0 et
- détermination d'une troisième position M dans la distance de cheminement en appliquant la formule M = Ff + (Fs - Ff)/2 et
- positionnement de l'intervalle I, de manière à ce que la position M forme le milieu de l'intervalle I.

5. Système de néphélométrie suivant la revendication 4, comprenant au moins deux, de préférence au moins 16, d'une manière particulièrement préférée au moins 32 positions de réception pour respectivement une cellule de mesure.

6. Système de néphélométrie suivant la revendication 5, dans lequel les au moins deux positions de réception pour respectivement une cellule de mesure sont disposées suivant une trajectoire circulaire.

7. Système de néphélométrie suivant l'une des revendications 4 à 6, dans lequel la source lumineuse, le diaphragme et le photodétecteur sont mobiles sur une trajectoire circulaire relativement à la au moins une position de réception de la cellule de mesure.

8. Système de néphélométrie suivant l'une des revendications 4 à 6, dans lequel la au moins une position de réception d'une cellule de mesure est mobile par rapport à la source lumineuse, au diaphragme et au photodétecteur.

9. Système de néphélométrie suivant l'une des revendications 4 à 8, dans lequel la au moins une position de réception est appropriée à la réception d'une cellule de mesure de section transversale ovale ou circulaire.

10. Appareil automatique d'analyse comportant un système de néphélométrie suivant l'une des revendications 4 à 9.

11. Appareil automatique d'analyse suivant la revendication 10, comprenant, en outre, un récipient de réception d'une pluralité de cellules de mesure en vrac, un dispositif d'individualisation des cellules de mesure et un dispositif de mise en position d'une cellule de mesure individuelle dans la au moins une position de réception de l'unité optique du système de néphélométrie.
